Europäisches Patentamt

European Patent Office

Office européen des brevets

(11) Numéro de publication : **0 447 297 A1**

(12) **DEMANDE DE BREVET EUROPEEN**

(21) Numéro de dépôt : **91400612.7**

(22) Date de dépôt : **06.03.91**

(51) Int. Cl.⁵ : **A61K 9/52, A61K 9/38**

(30) Priorité : **08.03.90 FR 9002972**

(43) Date de publication de la demande :
**18.09.91 Bulletin 91/38**

(84) Etats contractants désignés :
**AT BE CH DE DK ES FR GB GR IT LI LU NL SE**

(71) Demandeur : **RHONE-POULENC NUTRITION ANIMALE**
**Rue Marcel Lingot**
**F-03600 Commentry (FR)**

(72) Inventeur : **Ardaillon, Pierre**
**10 Impasse Beau-Vallon**
**F-69800 Saint-Priest (FR)**
Inventeur : **Franzoni, Christine**
**14 Quai de la Pêcherie**
**F-69001 Lyon (FR)**
Inventeur : **Prud'Homme, Christian**
**19 rue Commandant Faurax**
**F-69006 Lyon (FR)**

(74) Mandataire : **Le Pennec, Magali et al**
**RHONE-POULENC RORER SA, Direction des Brevets, 20 Avenue Raymond Aron**
**F-92160 Antony (FR)**

(54) Procédé d'enrobage par de la zeine de principes actifs.

(57)     L'invention concerne un nouveau procédé d'enrobage de principes actifs médicamenteux et/ou alimentaires qui consiste à pulvériser sur ceux-ci une émulsion ou une dispersion aqueuse de zéine.

EP 0 447 297 A1

## PROCEDE D'ENROBAGE PAR DE LA ZEINE DE PRINCIPES ACTIFS

La présente invention concerne un procédé d'enrobage de principes actifs par des formulations contenant de la zéïne. Elle concerne plus particulièrement un procédé d'enrobage par une émulsion ou une suspension aqueuse de zéïne, de principes actifs médicamenteux et/ou alimentaires.

Il est connu depuis longtemps d'enrober des principes actifs par de la zéïne éventuellement mélangée avec une substance hydrophobe telle que l'acide stéarique et/ou un polymère non hydrosoluble tel que l'éthylcellulose. Ces enrobages sont particulièrement décrits en ce qui concerne l'alimentation animale dans les demandes de brevets publiées sous les numéros EP 321 337 et EP 188 953.

Selon ces demandes on enrobe des particules de méthionine et/ou de lysine par la technique du lit fluidisé à l'aide d'une solution de zéïne dissoute dans un mélange de dichlorométhane et d'éthanol (50/50). Cette technique permet une pulvérisation facile de la zéïne mais présente l'inconvénient d'introduire une quantité importante de solvants halogénés, environ 20 à 50 g de dichlorométhane pour 1 g de zéïne. La zéïne est un produit naturel ce qui présente un avantage du point de vue écotoxicité considérable. Il est regrettable de devoir introduire concommitamment au cours du procédé une quantité importante d'une classe de produits dont l'écotoxicité n'est pas aussi remarquable c'est à dire les solvants halogénés.

La présente invention a permis d'enrober à l'aide de zéïne des principes actifs tels que les acides aminés ou les vitamines en évitant au maximum l'usage de solvants organiques non compatibles avec les milieux biologiques.

En effet la présente invention concerne un procédé d'enrobage par de la zéïne de principes actifs médicamenteux ou alimentaires par pulvérisation sur ceux-ci d'une émulsion ou d'une dispersion aqueuse de zéïne. Les principes actifs médicamenteux ou alimentaires à enrober se présentent de préférence sous forme de granulés ayant un diamètre compris entre 0,3 et 3 mm.

L'utilisation d'une émulsion aqueuse permet une économie importante dans la mise en oeuvre du procédé car elle évite l'utilisation d'installations de récupération de solvants coûteuses du point de vue sécurité et investissement. Elle permet en plus une productivité nettement améliorée par rapport aux procédés de l'art antérieur utilisant des solvants.

Les principes actifs médicamenteux sont choisis notamment parmi les vitamines, les antibiotiques, les composés antiparasitaires, les hormones. Les principes actifs alimentaires sont choisis notamment parmi les acides aminés essentiels supposés limitants tels que la méthionine et/ou la lysine ou ses dérivés et/ou le tryptophane.

On peut adjoindre à ces principes actifs des charges, éventuellement lamellaires, qui facilitent le délitement du granulé dans le tractus digestif. Ces charges sont notamment choisies parmi les charges PH sensibles ou non telles que par exemple : le talc et/ou la silice et/ou les carbonates et/ou les polyphosphates complexes tels que à base de $Na_2O$, CaO, $P_2O_5$ et $Al_2O_3$. On peut également adjoindre à ces principes actifs des agents liants choisis parmi les acides ou les esters gras, la cellulose (par exemple celle commercialisée sous la dénomination Avicel) ou ses dérivés, notamment l'éthylcellulose, la carboxyméthylcellulose, ainsi que les copolymères basiques. L'ensemble du ou des principes actifs et des additifs forment le coeur du granulé qui est enrobé par de la zéïne pulvérisée sous forme d'une émulsion aqueuse.

Ces principes actifs médicamenteux ou alimentaires contenant éventuellement un des additifs cités précédemment et enrobés de zéïne sont particulièrement intéressants pour l'alimentation des ruminants car ils ne sont pas dégradés lors du transit dans le rumen et sont libérés dans la caillette et/ou l'intestin.

L'enrobage est réalisé par pulvérisation d'une émulsion ou d'une dispersion aqueuse de zéïne. L'émulsion d'enrobage peut aussi contenir des additifs tels que ceux mentionnés précédemment pour le coeur, ainsi que des agents antistatiques, des agents plastifiants, des colorants ou des agents d'apétence.

On préfère utiliser une dispersion aqueuse contenant la zéïne et une substance hydrophobe et contenant éventuellement un polymère non hydrosoluble. La substance hydrophobe est choisie de préférence parmi les acides gras contenant 12 à 22 atomes de carbone tels que par exemple l'acide stéarique ou l'acide béhénique. L'émulsifiant peut être choisi parmi les esters d'acides gras ou les sels d'acides gras. L'émulsifiant peut dans ce dernier cas être créé "in situ" par salification de l'acide gras au moyen d'une base choisie parmi les hydroxydes alcalins et d'ammonium.

Le polymère non hydrosoluble est avantageusement choisi parmi les ethers et les esters de cellulose non hydrosolubles tels que l'éthylcellulose, l'acétate de cellulose, le propionate de cellulose, l'acétobutyrate de cellulose.

Cette émulsion ou cette dispersion est réalisée par mélange d'une solution de zéïne dans une faible quantité de solvant organique et d'une solution aqueuse contenant un émulsifiant. L'émulsion est préparée par mélange d'une phase aqueuse majoritaire et d'une phase organique. D'un point de vue mise en oeuvre, on préfère que la phase aqueuse représente 95 à 50 % en volume et la phase organique représente 5 à 50 % en volume. La phase aqueuse contient l'émulsifiant et/ou un de ses précurseurs permettant la formation "in situ" de l'émulsifiant. Ainsi elle contient notamment 0 à 10 % en poids d'un émulsifiant, choisi comme mentionné précédemment, parmi les esters ou les sels d'acides gras et/ou elle contient 0 à 1 % en poids d'un hydroxyde alcalin ou d'ammonium. Il est évident que pour que l'émulsion se forme, au moins l'un des deux groupes de constituants, soit l'émulsifiant, soit l'hydroxyde alcalin ou d'ammonium et l'acide gras doit être présent. La quantité minimale d'émulsifiant additioné ou formé "in situ", calculée sur la base de l'hydroxyde de sodium est de 0,2 % en poids.

La phase organique est obtenue par dispersion ou mise en solution d'un mélange solide ayant de préférence la constitution pondérale suivante:

– zéïne 10 à 100 %
– une ou plusieurs substances hydrophobes 0 à 90 %
– un ou plusieurs polymères non hydrosolubles 0 à 90 % dans une composition liquide constituée en volume de:
– 100 à 40 % de solvant organique ou de mélange de solvants organiques
– 0 à 60 % d'eau.

Le solvant organique ou le mélange de solvants organiques utilisé pour former la phase organique est choisi parmi les alcools linéaires ou ramifiés contenant au moins quatre atomes de carbone et les solvants mixtes alcool-solvants halogénés tels que par exemple le tetrachloroéthane, le trichloroéthane, le dichloroéthane, le chloroforme, le dichlorométhane.

Le pourcentage pondéral du mélange solide et de la composition liquide est compris notamment dans les limites suivantes:

– mélange solide 85 à 20 %
– composition liquide 15 à 80 %.

La phase organique, obtenue après dispersion ou mise en solution du mélange solide dans la composition liquide est mélangée avec la phase aqueuse préparée précédemment. Il est évident que lorsque la phase organique ne contient pas d'acide gras, la phase aqueuse contiendra obligatoirement un émulsifiant, par contre lorsque la phase organique contient au moins un acide gras, la phase aqueuse peut contenir un hydroxyde alcalin ou d'ammonium et/ou un émulsifiant.

L'émulsion est réalisée indifféremment par introduction de la phase organique dans la phase aqueuse ou inversement.

Avant pulvérisation de l'émulsion, celle ci est maintenue liquide à une température comprise entre la température ambiante et 90°C et de préférence entre 50 et 70°C. Elle est pulvérisée sur un lit de granulés à enrober par la technique du lit fluidisé de type par exemple WURSTER. Cette technique est par exemple décrite dans les brevets US 2 799 241 et EP 188 953.

Les granulés obtenus après enrobage sont utilisés pour l'alimentation ou le traitement médicamenteux des ruminants.

Leur préparation sera plus complètement décrite à l'aide des exemples suivants qui ne doivent pas être considérés comme limitatifs de l'invention

## EXEMPLE 1

On dissout 88 g d'acide stéarique (PF = 66,9°C, indice d'acide 199) acide PRIFRAC® (commercialisé par UNICHEMA) et 14 g de zéïne F 4000 (commercialisé par BENZIAN) avec 8 g d'éthylcellulose N 22 (commercialisé par HERCULES) dans 60 g de mélange contenant 100 parts de N-butanol et 20 parts d'eau.

On chauffe cette solution à 72°C et on l'ajoute en 7,5 minutes à une solution, agitée au moyen d'une turbine de type Polytron, de 495 cm3 d'eau et 4,2 ml de soude aqueuse à 10% (p/v). On obtient ainsi une émulsion qui est fluide et homogène à une température supérieure ou égale à 65°C.

Selon la technique du lit fluidisé avec une cuve équipé d'un système WURSTER, on enrobe 350 g de méthionine, préalablement granulée sous forme de particules sphériques titrant 98% dont le diamètre moyen est compris entre 0,63 et 0,8 mm par l'émulsion obtenue précédemment.

L'émulsion, maintenue à 70°C, est pulvérisée en 48 minutes.

On obtient ainsi 437 g de granulés enrobés titrant 77,5 % de méthionine.

## Test in vitro

La libération de la méthionine contenue dans les granulés obtenus est examinée, dans des conditions déterminées, en agitant une quantité connue de granulés dans un milieu tamponné maintenu à PH constant à une température de 40°C. On compare les vitesses de libération d'un échantillon soumis à différents PH.

On place 8 g de granulés contenant 77,5 % de méthionine préparés selon le mode opératoire précédent dans 1000 ml d'un milieu maintenu à PH6, les granulés ont libérés 3,3 % de méthionine après 24 heures.

## Test in vivo

On détermine la résistance des granulés à un séjour dans le rumen, chez le mouton en mesurant la quantité totale d'azote disparue des sachets.

Des sachets de nylon (maille carrée de 46 microns) contenant chacun environ 3g de granulés enrobés pesés exactement, sont incubés pendant 12 heures dans le rumen de deux moutons à raison de deux sachets par produit et par mouton.

Après incubation dans le rumen les sachets sont lavés à l'eau froide puis lyophilisés pendant 48 heures. Les sachets sont pesés et la quantité de granulés ayant disparu est déterminée. Les résidus sont ensuite sortis des sachets et échantillonnés en double pour le dosage d'azote total.

Après 17 heures la quantité d'aminoacides libérée dans le rumen est de 2%.

EXEMPLE 2

On reproduit l'exemple 1 en remplaçant les 110 g du mélange d'acide stéarique/zéïne/éthylcellulose par 110 g de zéïne. Si on ajoute 10 % d'oléate de sodium par rapport à la matière sèche, on observe la formation d'une émulsion homogène.

EXEMPLE 3

On dissout à chaud (75-80°C) 156 g d'acide stéarique acide PRIFRAC® (commercialisé par UNICHEMA) et 24,8 g de zéïne F 4000 (commercialisé par BENZIAN) avec 14,2 g d'éthylcellulose N 22 (commercialisé par HERCULES) dans 106 g de mélange contenant 100 parts de N-butanol et 20 parts d'eau.

Dans un réacteur de 2 litres, placé dans un bain marie et équipé d'une turbine Polytron, on charge 877 cm$^3$ d'eau distillée additionnée de 7,5 cm$^3$ de solution d'hydroxyde de sodium à 10 %. Le contenu du réacteur est chauffé à 65°C. On coule en 2 minutes 30 secondes la solution organique dans la phase aqueuse, sous agitation ( Polytron 11000 tours par minute ). Après la fin de la coulée, l'émulsion est brassée pendant 1 minute 30 secondes environ.

Selon la technique du lit fluidisé avec une cuve équipé d'un système WURSTER, on enrobe 350 g de chlorhydrate de lysine, préalablement granulé sous forme de particules sphériques titrant 90,4% dont le diamètre moyen est de 0,8 mm.

L'émulsion, maintenue à 75°C, est pulvérisée en une heure 36 minutes.

On obtient ainsi des granulés enrobés titrant 61 % de chlorhydrate de lysine avec un taux d'enrobant de 32,5 %.

Ces granulés sont testés in vitro selon la même technique qu'à l'exemple 1. Le taux de libération de la lysine chlorhydrate à PH6 à 40°C est de:
– en 6 heures: 3 %
– en 24 heures: 21 %

**Revendications**

1 - Procédé d'enrobage par de la zéïne de principes actifs médicamenteux et/ou alimentaires caractérisé en ce que l'on pulvérise sur ce ou ces principes actifs une émulsion ou une dispersion aqueuse de zéïne.

2 - Procédé selon la revendication 1 caractérisé en ce que l'émulsion ou la dispersion aqueuse de zéïne est obtenue par mélange d'une phase organique, contenant la zéïne et éventuellement une substance hydrophobe et/ou un polymère non hydrosoluble et d'une solution aqueuse contenant un émulsifiant ou un de ses précurseurs permettant la formation "in situ" de l'émulsifiant.

3 - Procédé selon la revendication 1 caractérisé en ce que le principe actif est un acide aminé choisi parmi la méthionine et/ou la lysine ou un de ses dérivés.

4 - Procédé selon la revendication 2 caractérisé en ce que la substance hydrophobe est choisie parmi les acides gras contenant 12 à 22 atomes de carbone et de préférence est l'acide stéarique.

5 - Procédé selon la revendication 2 caractérisé en ce que le polymère non hydrosoluble est choisi parmi les éthers et les esters de cellulose non hydrosolubles.

6 - Procédé selon la revendication 2 caractérisé en ce que l'émulsifiant est choisi parmi les sels et les esters d'acides gras.

7 - Procédé selon la revendication 2 caractérisé en ce que le précurseur de l'émulsifiant permettant la formation "in situ" de ce dernier est choisi parmi les hydroxydes alcalins ou d'ammonium.

8 - Procédé selon la revendication 1 caractérisé en ce qu'on ajoute au principe actif une charge choisie parmi les charges PH sensibles ou non telles que : le talc et/ou la silice et/ou les carbonates et/ou les polyphosphates complexes tels que à base de $Na_2O$, $CaO$, $P_2O_5$ et $Al_2O_3$.

9 - Procédé selon les revendications 2 et 8 caractérisé en ce que l'émulsion ou la dispersion contient en plus un ou plusieurs additifs choisis parmi les charges telles que définis à la revendication 8, des agents antistatiques, des agents plastifiants, des colorants et des agents d'apétence.

10 - Procédé selon l'une quelconque des revendications précedentes caractérisé en ce que l'on pulvérise sur des granulés de méthionine et/ou de lysine une émulsion constituée d'un mélange de zéïne, d'éthylcellulose, d'acide stéarique, de stéarate de sodium et d'eau.

11 - Procédé selon l'une quelconque des revendications précedentes caractérisé en ce que l'émulsion est préparée par mélange d'une phase aqueuse représentant 95 à 50 % en volume et d'une phase organique représentant 5 à 50 % en volume.

12 - Procédé selon la revendication 11 caractérisé en ce que la phase aqueuse contient 0 à 10 % en poids d'un émulsifiant selon la revendication 6 et/ou elle contient 0 à 1 % en poids d'un hydroxyde alcalin ou d'ammonium, la somme des deux représentant au moins 0,2 % en poids d'équivalent d'hydroxyde de sodium.

13 - Procédé selon la revendication 11 caractérisé en ce que la phase organique est obtenue par dispersion ou mise en solution d'un mélange solide ayant la constitution pondérale suivante:

– zéïne 10 à 100 %

– une ou plusieurs substances hydrophobes 0 à 90 %

– un ou plusieurs polymères non hydrosolubles 0 à 90 % dans une composition liquide contenant en volume:

– 100 à 40 % de solvant organique ou de mélange organique

– 0 à 60 % d'eau.

**14 -** Procédé selon la revendication 11 caractérisé en ce que le pourcentage pondéral du mélange solide et de la composition liquide est compris dans les limites suivantes:

– phase solide 85 à 20 %

– composition liquide 15 à 80 %.

**15 -** Procédé selon la revendication 13 caractérisé en ce que le solvant organique ou le mélange organique est choisi parmi les alcools linéaires ou ramifiés contenant au moins quatre atomes de carbone et les solvants mixtes alcool-solvants halogénés.

Office européen
des brevets

**RAPPORT DE RECHERCHE EUROPEENNE**

Numero de la demande

EP 91 40 0612

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | DERWENT FILE SUPPLIER, WPI, 1966, AN=66-19131F [00], Derwent Publications Ltd, Londres, GB; & JP-B-65 025 516 (TAISHO PHARMA CO., LTD) * Résumé * | 1,2,4-6 ,8,9,12 | A 61 K 9/52 A 61 K 9/38 |
| D,Y | EP-A-0 321 337 (RHONE POULENC SANTE) * Page 2, lignes 4-5,41-56; page 3, lignes 1-9; page 5, exemples 2,3; revendications * | 1-15 | |
| Y | EP-A-0 260 186 (RHONE POULENC SANTE) * Le document en entier * | 1-15 | |
| A | US-A-3 802 896 (E.B. WESTALL et al.) * Colonne 2, lignes 51-63; colonnes 5,6, examples 1,3; revendications 1,2,8-10 * | 1,2 | |

**DOMAINES TECHNIQUES RECHERCHES (Int. Cl.5)**

A 61 K
A 23 L

Le présent rapport a été établi pour toutes les revendications

| Lieu de la recherche | Date d'achèvement de la recherche | Examinateur |
|---|---|---|
| LA HAYE | 14-06-1991 | BOULOIS D.J-M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)